# EUROPEAN PATENT SPECIFICATION

(11) **EP 1 175 527 B1**
(45) Date of publication and mention of the grant of the patent: **13.08.2003**
(21) Application number: 00925448.3
(22) Date of filing: 13.04.2000
(51) Int. Cl.: D06N 3/00, D06N 7/00, A61L 15/00, A61F 13/00

(54) **PROCESS FOR COATING A PERFORATED SUBSTRATE**
VERFAHREN ZUM BESCHICHTEN VON PERFORIERTEN SUBSTRATEN
PROCEDE DE REVETEMENT D'UN SUBSTRAT PERFORE

(30) Priority: 23.04.1999 GB 9909349
(43) Date of publication of application: 30.01.2002
(73) Proprietor: First Water Limited, Marlborough, Wiltshire SN8 2RB (GB)
(72) Inventor: MUNRO, Hugh, Semple, Chipping Camden, Warwickshire GL55 6QT (GB); LAWRENCE, Steven, John, Coventry CV6 2JB (GB)
(74) Representative: Brown, David Leslie
(86) International application number: GB0001415
(87) International publication number: WO00065143

(56) References cited:
- EP-A- 0 676 457
- WO-A-93/19709
- WO-A-97/42985
- FR-A- 2 783 412
- US-A- 4 661 099
- US-A- 4 838 253
- US-A- 4 921 704
- US-A- 5 352 508

## Description

This invention relates to a process for coating a perforated substrate with a gel and to coated perforated substrates obtainable by the process.

### Background

Gel coated perforated substrates are known for use in a variety of consumer care applications, for example as wound dressings. The problem with existing methods for their preparation is that it is difficult to ensure that the perforations in the substrate are not occluded by the gel.

Furthermore known processes (see for example that used in US 5352508 (Cheong) to produce a wound dressing) encapsulate the perforated substrate within the gel. This is a problem because both sides of the substrate encapsulated by the gel are tacky and will need protecting with additional layers which adds complication and cost to the production process. In use it can be disadvantageous to have gel on both sides of the substrate. For example where the encapsulated substrate is to be used as a wound dressing which contacts the wound directly, it is not possible to have removable surface dressings (e.g. to absorb the exudate from the wound) on top of the wound dressing. This is because when the surface dressings are removed, they will disturb the wound dressing itself which potentially would interfere with the healing process.

It is an object of this invention to provide an improved process for coating a perforated substrate with a gel whereby substantially all of the perforations of the coated substrate produced are not occluded and wherein preferably only one side of the substrate is coated by the gel.

### Summary of the Invention

According to the present invention there is provided a process for coating a perforated substrate with a gel without substantial occlusion of the perforations, which process comprises:
(i) forming (e.g. by extrusion) a layer of a liquid pregel mixture, comprising one or more monomers, on a web coated with a coating having a surface energy less than the surface energy of the liquid pregel mixture;
(ii) contacting the perforated substrate with the liquid pregel mixture layer; and
(iii) curing the liquid pregel mixture.

The web is preferably removed after at least an initial partial curing of step (iii).

The term "perforated substrate" used herein refers to any substrate (including a portion thereof) having perforations (foramina). In particular, it includes woven and non-woven mesh fabrics. Further details of suitable perforated substrates are given below.

The term "without substantial occlusion of the perforations" used herein refers to at least about 70%, typically at least about 80%, more preferably at least about 90%, most preferably at least about 95%, of the perforations in the substrate being unoccluded.

In a particularly preferred embodiment, the invention provides a process for coating with a gel at least a portion of at least one major face of a planar perforated substrate having first and second major faces, and at least partially coated perforated substrates prepared thereby.

The web used in the invention is generally coated with any coating generally known in the art to reduce the surface energy of the material to which it is applied. For example a suitable coating is silicone, polyethylene, polyvinyl fluoride or polytetrafluroethylene (PTFE) (for example that sold under the trade name Teflon). The web is preferably made from paper, polyester, polyolefin or any other material that can act as a support for a low energy surface coating.

The coated perforated substrate according to the invention is useful in a wide variety of applications, including consumer care applications especially where the permeability or breathability of the perforated substrate is useful such as for cosmetic applications, e.g. for the attachment of wigs or toupees, or for therapeutic applications, e.g. as wound dressings, transdermal drug delivery, therapeutic patches or as electrodes (e.g. biomedical skin electrodes).

One advantage of the process according to the invention is that the perforations of the coated perforated substrate produced by the process are substantially unoccluded by gel. Without restricting the scope of the invention claimed, it is believed to be due to the combination of the contraction of the pregel after extrusion and the low surface energy of the web. This is because of the difference in surface energy between the pregel and the web as the pregel has a higher surface energy. The gel does not contract immediately because it is generally extruded in a relatively large amount. Thus it is not until the perforated substrate is applied on top of the extruded pregel that the pregel reticulates such that the perforations start to become free from the pregel.

The amount of the pregel extruded is generally from 0.01kg/m² to 3kg/m². The amount used will depend upon the intended purpose of the coated substrate. In general the amount should not be so small that the reticulation of the pregel on the substrate leads to the pregel becoming discontinuous with the formation of unconnected "islands" of pregel on the substrate. On the other hand the amount of pregel should not be so much that reticulation cannot occur. The coating weight of pregel at which these limitations are reached will depend upon a variety of factors including the viscosity of the pregel, the surface energy of the pregel and of the coating on the web, the size and number of the perforations in the substrate and the nature of the substrate itself.

The substrate/pregel/web assembly is then subjected to an initial cure which is found to further encourage the non-occlusion of the perforations. The web is then removed from the coated substrate. Optionally a final cure is applied, if necessary, either before or after the web is removed from the coated substrate. Conventional means are used to cure the gel e.g. heat, irradiation by UV light or electron beams.

A release sheet is optionally applied to the (or each) coated face of the substrate to support and protect it. The release sheet is generally either made of plastic or coated paper e.g. siliconised paper.

The perforated substrate used in the invention is generally any conventional substrate. The substrate is typically planar having first and second major faces, one (or, less preferably, both) of which is/are to be gel-coated. The substrate is generally either knitted, extruded, woven or non-woven. The smallest dimension of each perforation in the substrate is preferably from 0.5 to 5.0mm, more preferably from 1.0 to 3.0mm. The fibres are made from, for example, cotton, rayon, polyester, polyamide, polypropylene, polyamide and/or wool. The substrate, in the case of a patch for transdermal (iontophoretic) drug delivery or a biomedical skin electrode, may be electrically conductive.

It is found that the method of the present invention enables at least one major face of the perforated substrate to be satisfactorily coated with gel. without substantial occlusion of the perforation.

Generally the gel formed by the liquid pregel mixture used in the invention is any gel conventionally used to coat substrates, for example, to act as an adhesive. Preferably the gel formed by the pregel is a tacky gel such as a hydrogel or a xerogel. The gel is preferably a skin-compatible tacky hydrogel. Typically the pregel mixture used to form a hydrogel is an aqueous solution of one or more ionic monomers, optionally in association with a cross-linking agent.

In preferred embodiments the one or more ionic monomers will be acrylate based monomers selected for their ability to polymerise rapidly in water. Where there is more than one monomer, they preferably have substantially the same molecular weight whereby in a mixture of the two the relative proportions may be varied without significantly altering the molar characteristics of the composition The one or more ionic monomers are preferably included in the composition in an amount by weight of from 1% to 95%, more preferably from 10% to 70%, most preferably from 15% to 60%.

Preferably the one or more ionic monomers are 2-acrylamido-2-methylpropane sulphonic acid or an analogue thereof or one of its salts, e.g. an alkaline metal salt such as a sodium, potassium or lithium salts; acrylic acid or an analogue thereof or one of its salts, e.g. an alkaline metal salt such as a sodium, potassium or lithium salt; and/or a polymerisable sulphonate or a salt e.g. an alkaline metal salt such as a sodium, potassium or lithium salt, of acrylic acid (3-sulphopropyl)ester or an analogue thereof. The term "analogue" in this context refers particularly to substituted derivatives of 2-acrylamido-2-methylpropane sulphonic acid, of acrylic acid or of acrylic acid (3-sulphopropyl) ester.

A particularly preferred ionic monomer is a sodium salt of 2-acrylamido-2-methylpropane sulphonic acid, commonly known as NaAMPS which is available commercially at present from Lubrizol as either a 50% aqueous solution (reference code LZ2405) or a 58% aqueous solution (reference code LZ2405A) and/or acrylic acid (3-sulphopropyl) ester potassium salt, commonly known as SPA. SPA is available commercially in the form of a pure solid from Raschig.

Conventional crosslinking agents are preferably used to provide the necessary mechanical stability and to control the adhesive properties of the hydrogel. Preferably a crosslinker is included in an amount of at least 0.05% by weight. Typical crosslinkers include tripropylene glycol diacrylate, ethylene glycol dimethacrylate, triacrylate, polyethylene glycol diacrylale (PEG400 or PEG600), methylene bis acrylamide.

It has also been found that the addition of a polyhydric alcohol (such as glycerol, sorbitol or polyethylene glycol) to the pregel enhances the solubilisation process. This pregel is then converted into a gel by a free radical polymerisation reaction. This may be achieved for example using conventional thermal initiators and/or photoinitiators or by ionizing radiation. Photoinitiation is a preferred method and will usually be applied by subjecting the pre-gel reaction mixture containing an appropriate photoinitiation agent, for example Irgacure 184 (which is made by Ciba), to UV light after it has been spread or coated as a layer on siliconised release paper or other solid substrate. The processing will generally be carried out in a controlled manner involving a precise predetermined sequence of mixing and thermal treatment or history.

Features or characteristics of importance include the adhesive properties of the hydrogel which depend not only on the nature of the monomers used to form the hydrogel but also on the manner of processing, nature of plasticiser used and of added electrolyte (if any). These features, as well as the possible use of interpenetrants and other additives are discussed below.

### Adhesion

The performance of hydrogels as pressure sensitive adhesives is related to the surface energetics of the adhesive and of the adherend (for example mammalian skin) and to the viscoelastic response of the bulk adhesive. The requirement that the adhesive wet the adherend to maximise the work of adhesion is well known. This requirement is generally met when the adhesive has a similar or lower surface energy to the adherend. The viscoelastic properties, in particular the elastic or storage modulus (G') as measured by dynamic mechanical testing at low frequency (approximately 0.01 to 1Hz) and high frequency (100 to 1000Hz) have been related to the wetting/creep behaviour and peel/quick stick properties respectively. The choice, assembly and processing of the ingredients of the hydrogel adhesive is usually targetted at making a material with a balance of properties suitable for pressure sensitive adhesive applications. We have found that hydrogels with suitable surface energetics (for example 20-50x10⁻³ N/m (20 to 50 dynes/cm)) will function as useful adhesives capable of subsequent conversion into a product if the elastic moduli are within the ranges 500 to 20,000 Pa at a frequency of ca 1Hz, temperature 20° to 40° Celsius and 1000 to 100,000 Pa at a frequency of 100 Hz. A balance between the quantities and nature of polymer, plasticiser and the degree of crosslinking/entanglement has to be achieved.

### Plasticiser

The pregel for use in producing a hydrogel generally comprises, in addition to one or more ionic monomers, an aqueous plasticising medium and, optionally, additional electrolyte. It has been found that the activity of the water together with the rheological properties of the hydrogels generally need to be controlled to produce optimum pressure sensitive adhesive properties. One preferred feature of the preferred pregel used in the invention to form a hydrogel is that to achieve the desired adhesive properties the final amount of water required in the hydrogel is present in the pregel prior to gellation, i.e. no water is added to or removed from the hydrogel after manufacture. The pregel preferably comprises from 3% to 40% of water by weight of the mixture. The water acts both as a solvent for the monomers and as a plasticiser.

The aqueous plasticising medium optionally additionally comprises a polymeric or non-polymeric polyhydric alcohol (such as glycerol), an ester derived therefrom and/or a polymeric alcohol (such as polyethylene oxide). Glycerol is the preferred plasticiser. An alternative preferred plasticiser is an ester derived from boric acid and a polyhydric alcohol (such as glycerol). The pregel preferably comprises from 10% to 50%, preferably from 10% to 45%, of plasticiser (other than water) by weight of the mixture.

Plasticisers are generally used in the invention to control adhesive properties. Whilst the presence of glycerol or other polyhydric alcohols in other reported formulations has been quoted to provide humectant properties to the hydrogel, it has been discovered that the most important parameter to preventing water loss is the activity of the water within the hydrogel which in turn depends on the nature and proportions of the other components and manner of processing. The water activity of the hydrogel can be measured using impedance methods with devices such as the Rotronic AWVC (manufactured by Rotronic). The activity of water may also be determined by placing the hydrogel in environments of controlled humidity and temperature and measuring the changes in weight. The relative humidity (RH) at which the hydrogel does not change weight corresponds to the activity of water in the gel (%RH/100). The use of saturated salt solutions to provide the appropriate environmental conditions is well known. All hydrogels directly exposed to relative humidities less than that corresponding to the activity of water will be thermodynamically allowed to lose water. Exposure to greater relative humidities and the gel will gain weight. Water activity in the hydrogel is primarily dependent on the water content and the nature of the polymeric components and the way in which they are processed. Water activity has been shown to have a better correlation with the growth of bacteria and moulds than water content. It has been found that organisms struggle to grow at water activities less than 0.8. Enzyme activity has also been reported to decrease significantly below activity of 0.8.

One advantage of using hydrogels is that when they have water activities from 0.4 to 0.85, preferably from 0.65 to 0.8 and more preferably from 0.7 to 0.8, they are adhesive to dry skin. This is because they have a greater tendency to wet (i.e. donate water to the skin) rather than to extract. These materials do not encourage the growth of microbial agents and they can be sterilised. Hydrogels based on the curing of ionic monomers are preferred as they enable a greater control of the activity of water. For materials with requirements for higher water activities, e.g. from 0.75 to 0.85, monomers which are potassium salts are preferred, e.g. SPA, K AMPS, and K acrylate.

### Interpenetrants

Hydrogels based on interpenetrating polymer networks (IPN) are well known. As IPN has been defined as a combination of two polymers, each in network form, at least one of which has been synthesised and/or crosslinked in the presence of the other. As will be appreciated, this combination will generally be a physical combination rather than a chemical combination of the two polymers. IPN systems may be described by way of example as follows:

Monomer 1 is polymerised and crosslinked to give a polymer which is then swollen with monomer 2 plus its own crosslinker and initiator.

If only one polymer in the system is crosslinked the network formed is called a semi-IPN. Although they are also known as IPN's, it is only if there is total mutual solubility that full interpenetration occurs. In most IPN's there is, therefore, some phase separation but this may be reduced by chain entanglement between the polymers. It has also been reported that semi IPN's can be made in the presence of carrier solvents (for example water in the case of hydrophilic components).

It has been found that polymerising and crosslinking water soluble monomers in the presence of water soluble polymers, water and polyhydric alcohols produces hydrogel materials with enhanced rheological and consequently adhesive properties.

Suitable water soluble polymers for the formation of semi IPN's include poly (2-acrylamido-2-methylpropane sulphonic acid) or one of its salts and its copolymers, poly (acrylic acid-(3-sulphopropyl) ester potassium salt), copolymers of NaAMPS and SPA, polyacrylic acid, polymethacrylic acid. polyethylene oxide, polyvinyl methyl ether, polyvinyl alcohol, polyvinylpyrrolidone, its copolymers with vinyl acetate, dimethylaminoethyl methacrylate, terpolymers with dimethylaminoethyl methacrylate and vinylcaprolactam, polysaccharides such as gum arabic, karaya gum, xanthan gum, guar gum, carboxymethyl cellulose (CMC), NaCMC, hydroxypropylmethyl cellulose (HPMC), hydroxyethyl cellulose (HEC) or combinations thereof.

The amount of interpenetrant polymer used will be dependent on the mechanical and rheological properties required as well on consideration of processing conditions. If the interpenetrant polymer used increases the viscosity of the pre-gel mix beyond 5 Pa.s (5000 centipoise) we have found that the monomers do not polymerise and crosslink on an acceptable time scale (should be less than 60 seconds, preferably less than 10 seconds). The viscosity depends on the nature and molecular weight of the interpenetrant and the nature of pre-gel processing.

Of the natural polysaccharides, gum arabic is usually preferred due to its cold water solubility and lesser effect on viscosity compared with, for example, karaya gum. A higher concentration of gum arabic than karaya may therefore be used if desired, enabling a wider control of hydrogel properties. It has also been found that the processing steps for assembling the pre-gel formulation can be critical with respect to the properties of the manufactured hydrogel. For a given formulation, if the components are assembled at 25 °C and cured different adhesive properties are obtained compared to those that have been heated to 70°C. Solutions containing natural polysaccharides become less opaque indicative of improved solubility. The activity of water in hydrogels prepared from heat treated pre-gels generally is lower than in non heat treated pre-gels.

### Other additives

Non-hydrophilic polymers may also be incorporated either in the presence or absence of interpenetrant polymers to form phase separated materials. The preparation of two phase composites consisting of a hydrophilic polymer containing an ionically conducting continuous phase and domains of a hydrophobic pressure sensitive adhesive which enhance adhesion to mammalian skin have been reported in U.S. Patent 5338490. The method of preparation described therein involved casting a mixture (as a solution and or suspension) consisting of the hydrophilic polymer containing phase and hydrophobic components onto a substrate and then removing the solvent. It has been found, however, that adhesive ionically conducting hydrogels may be better prepared by combining the hydrophobic polymer (preferably as an emulsion) with the components of the pre-gel reaction mixture and casting these onto a substrate and curing. In other words, there is no need to remove a solvent in order to form useful materials. Furthermore, the hydrophilic phase in addition to being a crosslinked network may also be an IPN or semi IPN.

It is believed that when hydrophobic polymers are incorporated in this way that the hydrophobic component segregates to the surface (as determined by Fourier transform infrared attenuated total reflectance spectroscopy, FTIR ATR, approximate sampling depth 0.5 microns) and that it is the amount of the hydrophobic component present in the surface that influences the adhesion to a wide variety of materials. The greater the amount of the hydrophobic component the greater the adhesion. In U.S. Patent 5338490 weight ratios of the hydrophilic phase to the hydrophobic phase of 60:1 to 8:1 were claimed. In hydrogel adhesives of between 100 to 2000 microns thick made in accordance with the present invention, ratios of hydrophilic to hydrophobic components ranging from 7:1 to 1:50 have been found to be preferable, especially when these ratios are present in the surface of the adhesive composition. In the process of the present invention, however, it may take up to 72 hours from the initial curing of the adhesive hydrogel for the segregation of the hydrophobic materials to the surface, as defined by the ATR sampling depth, to be complete.

Preferably, the hydrophobic pressure sensitive adhesive in such embodiments is selected from the group consisting of polyacrylates, polyolefins, silicone adhesives. natural or synthetically derived rubber base and polyvinyl ethers or blends thereof. Preferably the hydrophobic pressure sensitive adhesive in these embodiments is an ethylene/vinyl acetate copolymer such as that designated DM137 available from Harco or a vinyl acetate dioctyl maleate copolymer available from Air Products (sold under the trade name Flexbond 150). Those skilled in the art will also know that the molecular weight and comonomer ratios may be altered to control the properties of hydrophobic pressure sensitive adhesives. In general, the degree of surface segregation exhibited by such hydrophobic pressure sensitive adhesive (HPSA) will be dependent on factors such as composition of the HPSA, viscosity of the pre-gel mixture, temperature and rate of curing.

Additional functional ingredients may also incorporated in the pregels used in the invention, including antimicrobial agents (e.g. citric acid, stannous chloride) and, for drug delivery applications, pharmaceutically active agents, the latter being designed to be delivered either passively (e.g. transdermally) or actively (e.g. iontophoretically) through the skin.

The invention will be further described with reference to the following Example which should not be understood to limit the scope of the invention.

### EXAMPLE 1

To 57 parts of a 58% solution of the sodium salt of 2-acrylamido-2-methylpropane sulphonic acid (NaAMPS) (LZ2405A) 10 parts of a 58% solution of the potassium salt of 3-sulphopropyl acrylate (SPA) were added along with 5 parts potassium chloride and stirred until the potassium chloride has dissolved. This solution was then mixed with 30 parts glycerol for 30 minutes. To the latter solution were added 0.15 parts of a solution containing 20 parts of polyethylene glycol diacrylate (pEG600) (product of UCB Chemicals marketed under the trade name designation of Ebacryl 11) in which 6 parts of 1-hydroxycyclohexyl phenyl ketone (product of Ciba and marketed under the trade name designation of Irgacure 184) were dissolved. The so-formed pre-gel solution was then extruded through a 15cm (6 inch) width slot die onto a web of siliconised paper (weight 140 g/m², supplied by Coatec) moving at a rate of 5 metres per minute at a coat weight of 0.8kg/m². A 100% polyester mesh (sold under the trade name BG3486 by Brightwater) was laid on top and the assembly was cured by being passed under a medium pressure mercury arc lamp at a speed of 5 meters per minute. The residence time under the lamp was 4 seconds. On completion of curing and removal of the web the polyester mesh was found to be gel-coated on only one of its major surfaces, with more than 70% of the perforations being unoccluded by gel.

## Claims

1. A process for coating a perforated substrate with a gel without substantial occlusion of the perforations, which process comprises:
(i) forming a layer of a liquid pregel mixture, comprising one or more monomers, on a web coated with a coating having a surface energy less than the surface energy of the liquid pregel mixture;
(ii) contacting the perforated substrate with the liquid pregel mixture; and
(iii) curing the liquid pregel mixture.

2. Aprocess according to claim 1, wherein the layer of the liquid pregel mixture is formed by extrusion of the liquid pregel mixture onto the web.

3. A process according to claim 1 or claim 2, wherein the contacting of the perforated substrate with the liquid pregel mixture is achieved by applying the substrate to the pregel mixture on the web.

4. A process according to any one of the preceding claims, wherein the weight of liquid pregel mixture on the web is between about 0.01 to about 3 kg/m².

5. A process according to any one of the preceding claims, wherein at least some of the curing takes place while the liquid pregel mixture is in contact with both the perforated substrate and the web.

6. A process according to any one of the preceding claims, wherein at least some of the curing takes place while the liquid pregel mixture is in contact with the perforated substrate after removal of the web.

7. A process according to any one of the preceding claims, wherein the web comprises paper, polyester, polyolefin or any combination thereof.

8. A process according to any one of the preceding claims, wherein the coating of the web comprises silicone, polyethylene, polyvinyl fluoride, PTFE or any mixture or combination thereof.

9. A process according to any one of the preceding claims, wherein the perforated substrate is planar, having first and second major faces, and the process applies the gel to at least a portion of at least one major face of the substrate.

10. A process according to claim 9, wherein the planar perforated substrate comprises woven or non-woven fibres of cotton, rayon, polyester, polyamide, polypropylene, wool or any mixture or combination thereof.

11. A process according to any one ofthe preceding claims, wherein the one or more monomers comprise at least one acrylate based monomer.

12. A process according to any one of the preceding claims, wherein the liquid pregel mixture includes one or more crosslinking agents for the monomer(s).

13. A process according to any one of the preceding claims, wherein the liquid pregel mixture is an aqueous mixture, optionally including also at least one plasticising agent other than water.

14. A process according to claim 13, wherein the liquid pregel mixture includes from about 3% to about 40% by weight of water.

15. A process according to any one of the preceding claims, wherein the curing is performed by heat, ultra-violet irradiation, electron beam irradiation or any combination thereof.

16. A process according to any one of the preceding claims, wherein the gel is formed by polymerisation of one or more monomers, optionally in the presence of one or more crosslinking agents for the monomer(s).

17. A process according to claim 16, wherein only one side of the substrate is coated by the gel.

18. A process according to claim 17, wherein the gel coat is protected by a contacting release sheet.

19. A process according to any one of claims 16 to 18, wherein the substrate is, or forms part of, an article being an attachment tab for a wig or toupee, a wound dressing, a patch for transdermal drug delivery, a therapeutic patch or a biomedical skin electrode.

## Patentansprüche

1. Verfahren zum Beschichten eines perforierten Substrats mit einem Gel ohne beträchtliche Okklusion der Perforationen, wobei das Verfahren Folgendes umfasst:
(i) Bildung einer Schicht eines flüssigen Prägel-Gemisches, welches ein oder mehrere Monomere umfasst, auf einer Trägerbahn, die mit einer Beschichtung beschichtet ist, welche eine kleinere Oberflächenspannung aufweist als das flüssige Prägel-Gemisch;
(ii) Kontaktierung des perforierten Substrats mit dem flüssigen Prägel-Gemisch; und
(iii) Aushärten des flüssigen Prägel-Gemisches.

2. Verfahren nach Anspruch 1,
wobei die Schicht des flüssigen Prägel-Gemisches durch Extrusion des flüssigen Prägel-Gemisches auf die Trägerbahn gebildet wird.

3. Verfahren nach Anspruch 1 oder 2,
wobei die Kontaktierung des perforierten Substrats mit dem flüssigen Prägel-Gemisch durch Aufbringen des Substrats auf das Prägel-Gemisch auf der Trägerbahn erreicht wird.

4. Verfahren nach einem der vorhergehenden Ansprüche,
wobei das Gewicht des flüssigen Prägel-Gemisches auf dem Gewebe zwischen etwa 0,01 und etwa 3 kg/m² beträgt.

5. Verfahren nach einem der vorhergehenden Ansprüche,
wobei zumindest ein Teil des Aushärtens stattfindet, während sich das flüssige Prägel-Gemisch in Kontakt sowohl mit dem perforierten Substrat als auch mit der Trägerbahn befindet.

6. Verfahren nach einem der vorhergehenden Ansprüche,
wobei das Aushärten zumindest teilweise stattfindet, während sich das flüssige Prägel-Gemisch nach Entfernen der Trägerbahn in Kontakt mit dem perforierten Substrat befindet.

7. Verfahren nach einem der vorhergehenden Ansprüche,
wobei die Trägerbahn Papier, Polyester, Polyolefin oder eine Kombination davon umfasst.

8. Verfahren nach einem der vorhergehenden Ansprüche,
wobei die Beschichtung der Trägerbahn Silikon, Polyethylen, Polyvinyl Fluorid, PTFE oder eine Mischung oder Kombination davon umfasst.

9. Verfahren nach einem der vorhergehenden Ansprüche,
wobei das perforierte Substrat eben ausgebildet ist, erste und zweite Hauptflächen aufweist und wobei durch das Verfahren das Gel auf zumindest einen Teil zumindest einer Hauptfläche des Substrats aufgebracht wird.

10. Verfahren nach Anspruch 9,
wobei das ebene perforierte Substrat Gewebe oder Vlies aus Baumwolle, Rayon, Polyester, Polyamid, Polypropylen, Wolle oder eine Mischung oder Kombination davon umfasst.

11. Verfahren nach einem der vorhergehenden Ansprüche,
wobei das eine oder die mehreren Monomere zumindest ein Monomer auf Acrylatbasis umfassen.

12. Verfahren nach einem der vorhergehenden Ansprüche,
wobei das flüssige Prägel-Gemisch ein oder mehrere Vernetzungsmittel für das Monomer/die Monomere beinhaltet.

13. Verfahren nach einem der vorhergehenden Ansprüche,
wobei es sich bei dem flüssigen Prägel-Gemisch um ein wässriges Gemisch handelt, welches wahlweise auch zumindest ein anderes Plastifizierungsmittel als Wasser beinhaltet.

14. Verfahren nach Anspruch 13,
wobei das flüssige Prägel-Gemisch etwa 3 bis etwa 40 Gewichtsprozent Wasser beinhaltet.

15. Verfahren nach einem der vorhergehenden Ansprüche,
wobei das Aushärten mittels Hitze, UV-Strahlung, Elektron-Strahlung oder eine Kombination davon durchgeführt wird.

16. Verfahren nach einem der vorhergehenden Ansprüche,
wobei das Gel durch Polymerisation eines oder mehrerer Monomere gebildet wird, wahlweise bei gleichzeitigem Vorhandensein von einem oder mehreren Vernetzungsmitteln für das Monomer/die Monomere.

17. Verfahren nach Anspruch 16,
wobei lediglich eine Seite des Substrats mit dem Gel beschichtet wird.

18. Verfahren nach Anspruch 17,
wobei die Gel-Beschichtung mittels einer Kontaktabziehlage geschützt wird.

19. Verfahren nach einem der Ansprüche 16 bis 18,
wobei das Substrat Teil eines Artikels ist oder bildet, der ein Befestigungselement für eine Perücke oder ein Toupet, einen Wundverband, ein Pflaster für transdermale Medikamentenverabreichung, ein therapeutisches Pflaster oder eine biomedizinische Hautelektrode darstellt.

## Revendications

1. Procédé pour revêtir un substrat perforé d'un gel sans occlusion sensible des perforations, ce procédé consistant :
i) à former une couche d'un mélange liquide de prégel comprenant un monomère ou plusieurs monomères sur une nappe revêtue d'un revêtement ayant une énergie superficielle inférieure à l'énergie superficielle du mélange liquide de prégel ;
ii) à mettre le substrat perforé en contact avec le mélange liquide de prégel ;
iii) à durcir le mélange liquide de prégel.

2. Procédé suivant la revendication 1 dans lequel on forme la couche du mélange liquide de prégel par extrusion du mélange liquide de prégel sur la nappe.

3. Procédé suivant la revendication 1 ou 2, dans lequel on effectue la mise en contact du substrat perforé avec le mélange liquide de prégel en appliquant le substrat au mélange de prégel sur la nappe.

4. Procédé suivant l'une quelconque des revendications précédentes, dans lequel le poids du mélange liquide de prégel sur la nappe est compris entre environ 0,01 et environ 3kg/m².

5. Procédé suivant l'une quelconque des revendications précédentes, dans lequel au moins une partie du durcissement a lieu alors que le mélange liquide de prégel est en contact à la fois avec le substrat perforé et la nappe.

6. Procédé suivant l'une quelconque des revendications précédentes, dans lequel au moins une partie du durcissement a lieu alors que le mélange liquide de prégel est en contact avec le substrat perforé après enlèvement de la nappe.

7. Procédé suivant l'une quelconque des revendications précédentes, dans lequel la nappe comprend du papier, un polyester, une polyoléfine ou l'une de leur combinaison.

8. Procédé suivant l'une quelconque des revendications précédentes, dans lequel le revêtement de la nappe comprend un silicone, du polyéthylène, du poly (fluorure de vinyle) du PTFE ou l'un quelconque de leurs mélanges ou combinaisons.

9. Procédé suivant l'une quelconque des revendications précédentes, dans lequel le substrat perforé est plan en ayant des première et seconde faces principales et le procédé applique le gel à au moins une partie d'au moins une face principale du substrat.

10. Procédé suivant la revendication 9, dans lequel le substrat perforé plan comprend des fibres tissées ou non tissées de coton, de rayonne, de polyester, de polyamide, de polypropylène, de laine ou de l'un de leurs mélanges ou de combinaisons.

11. Procédé suivant l'une quelconque des revendications précédentes, dans lequel le monomère ou les monomères comprennent au moins un monomère à base d'acrylate.

12. Procédé suivant l'une quelconque des revendications précédentes, dans lequel le mélange liquide de prégel comprend un ou plusieurs agents de réticulation du monomère ou des monomères.

13. Procédé suivant l'une quelconque des revendications précédentes, dans lequel le mélange liquide de prégel est un mélange aqueux comprenant éventuellement aussi au moins un agent plastifiant autre que de l'eau.

14. Procédé suivant la revendication 13, dans lequel le mélange liquide de prégel comprend entre environ 3%, environ 40 % en poids d'eau.

15. Procédé suivant l'une quelconque des revendications précédentes, dans lequel le durcissement est effectué par de la chaleur, par un rayonnement ultraviolet, par un rayonnement par faisceau d'électrons ou par l'une quelconque de leurs combinaisons.

16. Procédé suivant l'une quelconque des revendications précédentes, dans lequel on forme le gel par polymérisation d'un ou de plusieurs monomères, éventuellement en la présence d'un ou plusieurs agents de réticulation pour le ou les monomères.

17. Procédé suivant la revendication 16, dans lequel seulement une face du substrat est revêtue du gel.

18. Procédé suivant la revendication 17, dans lequel le revêtement de gel est protégé par une feuille de détachement de contact.

19. Procédé suivant l'une quelconque des revendications 16 à 18, dans lequel le substrat est ou forme une partie d'un objet ayant une patte de fixation pour une perruque ou un faux toupet, un pansement de blessure, un timbre d'administration de médicament par voie transdermique, un timbre thérapeutique ou une électrode biomédicale pour la peau.
